Europäisches Patentamt

European Patent Office

Office européen des brevets

(11) Publication number: **0 198 094 B1**

# EUROPEAN PATENT SPECIFICATION

(45) Date of publication of patent specification: **18.09.91** (51) Int. Cl.5: **A61K 7/16**

(21) Application number: **85905422.3**

(22) Date of filing: **24.10.85**

(86) International application number:
**PCT/JP85/00593**

(87) International publication number:
**WO 86/02547 (09.05.86 86/10)**

The file contains technical information submitted
after the application was filed and not included in
this specification

(54) **TOOTH PASTE BINDER COMPOSITION.**

(30) Priority: **24.10.84 JP 223380/84**

(43) Date of publication of application:
**22.10.86 Bulletin 86/43**

(45) Publication of the grant of the patent:
**18.09.91 Bulletin 91/38**

(84) Designated Contracting States:
**DE FR GB**

(56) References cited:
**JP-A- 5 933 210**

**Hawleys Condensed Chemical Dictionary,
11th, ed., van Nostrand and Reinhold Co.,
New York, USA**

(73) Proprietor: **DAICEL CHEMICAL INDUSTRIES,
LTD.**
**1, Teppo-cho Sakai-shi**
**Osaka 590(JP)**

(72) Inventor: **FUKUI, Yoshitaka**
**500, Kamiyobe**
**Yobeku, Himeji-shi Hyogo 671-12(JP)**

(74) Representative: **Lehn, Werner, Dipl.-Ing. et al
Hoffmann, Eitle & Partner Patentanwälte Ar-
abellastrasse 4
W-8000 München 81(DE)**

## Description

TECHNICAL FIELD

The present invention relates to a composition suitable for use as a binder in toothpastes, especially in toothpastes filled in the tube. More specifically, the present invention relates to a composition that is prepared by mixing sodium carboxymethyl cellulose (hereunder abbreviated as CMC-Na) with an aqueous suspension of microfibrillated cellulose (hereunder abbreviated as MFC) and dehydrating the mixture.

BACKGROUND ART

While toothpastes are available in various forms, most of them are sold in the form of tube in the market solely because of convenience. A primary object of using toothpastes is to give a polishing effect, but modern toothpastes are also required to have diverse additional features such as good shape retention when they are squeezed from the tube onto a toothbrush, a good mouth feel during brushing, a satisfactory ability to be washed off the teeth easily, and a stability of quality during storage. In order to to meet these requirements, a number of chemicals are incorporated in toothpaste formulations.

The components in a formulation for toothpastes are roughly divided into solid components such as abrasive, and liquid components such as humectants and water. The toothpaste formulation also contains a binder so that the solid components are mixed with the liquid components in a stable state and that the toothpaste can be easily squeezed out of the tube and retain its shape on a toothbrush. The binder also influences the outer appearance of the toothpaste, the feeling it affords during brushing, its foamability, and the ease with which the toothpaste can be washed off the teeth.

Water-soluble polymers are hitherto used as a binder, and CMC-Na is most commonly used because of its high performance and stability in quality.

In overall consideration of performance parameters such as price, hygiene, prevention of water separation, outer appearance and mouth feel, CMC-Na is one of the best binders available today, but it is not completely satisfactory as a binder for use in toothpastes.

That is, the viscosity of CMC-Na is highly sensitive to temperature and co-existing electrolytes (most abrasives are electrolytes) and has a tendency to decrease at high temperatures or in the presence of ions.

If, under such conditions, CMC-Na is employed in an increased amount, tee resistance to the squeezing of the toothpaste from the tube will be increased to an undesirable level or the toothpaste may give a "gummy" feeling to the user. Natural polysaccharides such as carrageenan which are more stable to ions and temperature than CMC-Na are often used, but their characteristic odor or instability in quality prevent such natural polysaccharides from expanded use on an industrial scale.

The present assignee previously filed a patent application JP-A-59-33210 directed to a toothpaste composition incorporating MFC. MFC is prepared from a slurry of pulp fibers by beating it in an aqueous medium in accordance with the method disclosed in JP-A-56-100801 wherein the MFC is defined as a "microfibrillated cellulose". The MFC is usually obtained as an aqueous suspension. An aqueous suspension of MFC with a solids content within the range of from about 0.5 to 6% is a paste-like stable dispersion of MFC.

The aqueous suspension of MFC has a high water retaining ability and imparts viscosity that is resistant to temperature and co-existing ions. If it is used as a binder in combination with CMC-Na, a toothpaste having superior performance in many respects such as shape retention, mouth feel and washability can be prepared.

However, as already mentioned, the aqueous suspension of MFC has a solids content of not more than a few percent and the greater part of it is made of water, this putting limits on the amount in which the aqueous suspension of MFC can be incorporated in a toothpaste formulation as a binder.

DISCLOSURE OF THE INVENTION

A primary object, therefore, of the present inveniton is to provide a binder that is composed of a combination of MFC with CMC-Na and which is suitable for use in a toothpaste. The viscosity of the aqueous suspension of MFC is not highly sensitive to temperature or co-existing ions so that said aqueous suspension of MFC will, when combined with CMC-Na, serve as a binder that is free from the defects of CMC-Na.

The present invention provides a composition useful in imparting viscosity to a toothpaste, said composition being prepared by adding a suspension of microfibrillated cellulose to sodium carboxymethyl cellulose in an amount ranging from 30 to 600 % of the cellulose solids content in the aqueous suspension of MFC, homogeneously mixing and finally dehydrating the mixture.

The aqueous suspension of MFC can be added to a toothpaste composition, but, as described above, the water present in a large volume puts limits on the amount in which said suspension can be effectively incorporated in the toothpaste. It is of course possible to remove the water by drying the aqueous suspension of MFC, but in this case, the cellulose becomes keratose and the dried MFC will not retain the initial viscosity even if it is re-dispersed in water. On the other hand, if a homogeneous composition prepared by adding a water-soluble polymer to a suspension of microfibrillated cellulose is dried, a dehydrated composition having improved re-dispersibility can be obtained. This method is disclosed in JP-A-60-186548

In accordance with the present invention, CMC-Na is used in an amount ranging from 30 to 600% of the cellulose solids content in the aqueous suspension of MFC and, by drying the mixture, the desired binder is obtained. The thus obtained binder may be mixed with water and other components of toothpastes in a manner similar to that employed for incorporating powders of ordinary water-soluble polymers, and as a result, a toothpaste formulation having the necessary viscosity is obtained.

The CMC-Na suitable for use in preparing the binder of the present invention is what is generally referred to as a "highly etherified CMC" whose degree of etherification is from 0.9 to 2.6. The CMC-Na whose degree of etherification is 0.9 or more has high resistance to viscosity changes resulting from the presence of ions or enzymes but has a tendency to exhibit poor shape retention for the same viscosity. It has been found that this defect of the highly etherified CMC-Na can be compensated for by mixing it with MFC.

Further, CMC-Na having a degree of etherification exceeding 2.6 is costly and does not exhibit a commensurate improvement in its performance.

The binder is generally incorporated in the toothpaste composition in an amount of 0.1 to 5 wt% of said composition, with the range of 1 to 1.5 wt% being preferred.

In the present invention, as components other than the binder, those components selected from among the customarily used compounds can be used depending upon the type of the toothpaste composition. Examples of the abrasive include calcium carbonate, an anhydrous or dihydrated product of dicalcium phosphate, tricalcium phosphate, calcium sulfate, aluminum hydroxide, alumina, insoluble sodium metaphosphate, silica, aluminosilicate, and calcium pyrophosphate. Other usable compounds include humectants such as glycerin, sorbitol, and propylene glycol; rinsing agents such as sodium lauryl sulfate and N-lauroyl sarcosine sodium; sweeteners such as saccharin sodium and stevioside; as well as preservatives, flavorings, colorants, and various other effective ingredients such as sodium chloride (as an astringent).

## BEST MODE FOR CARRYING OUT THE INVENTION

The present invention is explained below by reference to the following examples.

Preparation Example 1

To 100 g of an aqueous suspension of MFC with a cellulose solids content of 3% (viscosity, 8,000 cps, as measured at 25°C by a BL viscometer with rotor No. 4 rotating at 60 rpm), 3 g of CMC-Na with a degree of etherification of 2.4 (viscosity, 130 cps as a 1% aqueous solution, as measured at 25°C by a BL viscometer with rotor No. 2 rotating at 60 rpm) was added under agitation until a homogeneous mixture having CMC-Na dissolved in the aqueous phase of the MFC aqueous suspension was obtained. This mixture was dried with a blast of hot air (105°C) for 8 hours to obtain a dehydrated binder (No. 1). This binder was easily reconstituted into a homogeneous aqueous suspension by agitation in the presence of water. When the binder No. 1 was resuspended in water to a solids content of 1%, the following viscosity data were obtained (as measured at 25°C by a BL viscometer with rotor No. 2):

520 cps (60 rpm) and 2,990 cps (6 rpm).

This data shows the high thixotropy of the reconstituted aqueous suspension of the binder No. 1.

Preparation Example 2

A dehydrated binder (No. 2) was obtained as in Preparation Example 1 except that CMC-Na having a degree of etherification of 0.5 and a viscosity of 145 cps as a 1% aqueous solution was used. When the binder was reconstituted into an aqueous suspension with a solids content of 1%, the following viscosity data were obtained:

390 cps (60 rpm) and 1,570 cps (6 rpm).

The thixotropy of the binder No. 2 was as high as that of the binder No. 1.

Examples 1 and 2 and Comparative Example 1

Three samples of toothpastes were prepared using the same formulation except that only the binder was changed. Their simplified formulation is shown below (percentages are by weight):

Tricalcium phosphate            42.0%

Glycerin                        15.0%

Sorbitol (70%)                  15.0%

Water                           27.8%

Binder                           1.2%

The binders used are listed below. They were found to have similar values of viscosity as measured at a rotor speed of 60 rpm.

Example 1 .......   Binder No. 1

Example 2 .......   Binder No. 2

Comparative.......  CMC-Na (degree of etherifica-
Example 1

tion, 0.7; viscosity as a 1%

aqueous solution, 400 cps at

60 rpm, and 480 cps at 6 rpm)

Each of the three toothpaste samples was charged into polyethylene tube with a mouth having a diameter of 7 mm and a portion measuring 15 mm in length was squeezed therefrom onto a toothbrush. The ease with which the samples could be squeezed and the luster of the surface of the squeezed toothpaste were evaluated to be as follows:

Example 1 ≃ Example 2 ≫ Comparative Example 1.

The shape retention and outer appearance of the squeezed toothpaste samples were evaluated to be as follows:

Example 1 > Example 2 ≫ Comparative Example 1.

Example 3 and Comparative Example 2

Two samples of toothpastes were prepared using the same formulation except that only the binder was changed. Their complete formulation is shown below (percentages are by weight):

Dicalcium phosphate (dihydrate)      42.0%

Glycerin                             15.0%

4

| | |
|---|---|
| Sorbitol (70%) | 15.0% |
| Silicic anhydride | 3.0% |
| Stannous fluoride | 1.4% |
| Binder | 1.2% |
| Flavoring | 1.0% |
| Saccharin sodium | 0.2% |
| Sodium lauryl sulfate | 2.0% |
| Butyl paraoxybenzoate | 0.1% |
| Water | 20.0% |

The binder incorporated in the toothpaste sample of Comparative Example 2 was CMC-Na having a degree of etherification of 1.5 and a viscosity of 600 cps as a 1% aqueous solution (60 rpm). The binder used in the sample of Example 3 was a dehydrated composition prepared from a mixture of equal amounts of the same CMC-Na and MFC by the procedures employed in Preparation Example 1.

Each of the toothpaste samples was charged into a polyethylene tube having a mouth with a diameter of 7 mm and squeezed therefrom onto a toothbrush. The sample of Comparative Example 2 sagged slightly after squeezing out of the tube but the sample of Example 3 did not deform at all. The teeth were polished with a toothbrush carrying each of the two samples; the sample of Example 3 was superior in terms of both mouth feel and ease with which the toothpaste could be removed by washing with water.

The same results were obtained when the samples were squeezed onto a toothbrush after charging into the tube and heating at 40° C; the sample of Comparative Example 2 deformed slightly whereas the sample of Example 3 exhibited good shape retention.

INDUSTRIAL APPLICABILITY

A toothpaste incorporating the above described composition as a binder exhibits good shape retention and ease of squeezing, and washability. The toothpaste is also characterized by good mouth feel and possibility of extended storage. Additionally, the toothpaste withstands storage at high temperatures and permits incorporation of sodium chloride. The ease with which the toothpaste can be squeezed from the tube and the good shape retention after squeezing are significant features which distinguish said toothpaste from the conventional product.

Claims

1. A composition useful in imparting viscosity to a toothpaste, said composition being prepared by adding a suspension of microfibrillated cellulose to sodium carboxymethyl cellulose in an amount ranging from 30 to 600 % of the cellulose solids content in the aqueous suspension of MFC, homogeneously mixing and finally dehydrating the mixture.

Revendications

1. Composition utile pour conférer la viscosité à une pâte dentifrice, ladite composition étant préparée par addition d'une suspension de cellulose fibrillée à de la carboxyméthylcellulose sodique en une quantité comprise entre 30 et 600 % de la teneur en solides cellulosiques dans la suspension aqueuse de MFC, par mélange homogène et finalement déshydratation du mélange.

Patentansprüche

1. Zusammensetzung, geeignet um einer Zahnpasta Viskosität zu verleihen, wobei diese Zusammensetzung hergestellt wird, indem eine Suspension von microfibrillärer Zellulose zu Natriumcarboxymethylzellulose in einer Menge von 30 bis 600% des Zellulosefeststoffgehalts in der wässrigen Suspension von MFC hinzugefügt wird, homogen gemischt wird und letztendlich die Mischung dehydratisiert wird.